# EUROPEAN PATENT SPECIFICATION

(11) **EP 2 513 330 B1**
(45) Date of publication and mention of the grant of the patent: **08.03.2017**
(21) Application number: 10801235.2
(22) Date of filing: 16.12.2010
(51) Int. Cl.: C12Q 1/68

(54) **DIAGNOSTIC METHODS BASED ON SOMATICALLY ACQUIRED REARRANGEMENT**
DIAGNOSTISCHE VERFAHREN AUF DER BASIS VON SOMATISCH ERWORBENER NEUANORDNUNG
METHODES DE DIAGNOSTIC BASEES SUR UNE REORGANISATION GENOMIQUE ACQUISE SOMATIQUEMENT

(30) Priority: 17.12.2009 GB 0922006
(43) Date of publication of application: 24.10.2012
(73) Proprietor: Genome Research Limited, London NW1 2BE (GB)
(72) Inventor: CAMPBELL, Peter John, London, Greater London NW1 2BE (GB)
(74) Representative: Hayes, Emily Anne Luxford
(86) International application number: PCT/GB2010/052110
(87) International publication number: WO 2011/073665

(56) References cited:
- WO-A1-2009/057695
- WO-A2-2005/113824
- US-A1- 2008 166 704
- CAMPBELL PETER J ET AL: "Identification of somatically acquired rearrangements in cancer using genome-wide massively parallel paired-end sequencing." NATURE GENETICS, [Online] June 2008 (2008-06), pages 1-17, XP002622046 ISSN: 1546-1718 PMC -PubMed Central AUTHOR MANUSCRIPT Retrieved from the Internet: URL:http://www.ncbi.nlm.nih.gov/pmc/articl es/PMC2705838/pdf/ukmss-5231.pdf>
- MOSSE YAEL P ET AL: "Neuroblastomas have distinct genomic DNA profiles that predict clinical phenotype and regional gene expression." GENES, CHROMOSOMES & CANCER, vol. 46, no. 10, October 2007 (2007-10), pages 936-949, XP002622047 ISSN: 1045-2257
- HOSHINO MAKIKO ET AL: "Molecular analyses of cell origin and detection of circulating tumor cells in the peripheral blood in alveolar soft part sarcoma.", CANCER GENETICS AND CYTOGENETICS 15 APR 2009, vol. 190, no. 2, 15 April 2009 (2009-04-15), pages 75-80, ISSN: 1873-4456

## Description

Individualised health-care is a major goal for medicine in the next 5-10 years. Numerous advances are required to attain this objective, including the development of sensitive and specific biomarkers for measuring disease burden.

By way of one example, personalising cancer medicine depends upon the implementation of personalised diagnostics. Since cancer is, at its core, driven by somatic mutation, detailed genomic screening is likely to play a central role in facilitating individual therapeutic choices. As the range of drugs and other therapies for cancer continues to increase, there is an increasingly urgent need for sensitive and specific measures of disease burden to guide treatment regimens.

In haematological malignancies, there is routine quantification of residual disease levels through assays for recurrent genomic rearrangements. This has been made possible by the discovery that leukaemias are associated with characteristic genomic rearrangements that do not require either genome-wide screening or development of patient-specific assays. In solid tumours, however, methods for quantifying disease burden are less sensitive and less specific. Radiological imaging is routinely used for staging patients, but can only detect gross lesions >1cm in size, already representing many millions of cancer cells. Serum markers, such as PSA for prostate cancer, can be helpful, but are not available for many tumour types and frequently suffer from problems of non-specificity. Immunological detection of circulating tumour cells is sensitive down to 1 cancer cell in thousands of normal cells, but only detects cells present in the blood and can result in false positive calls from non-malignant cells expressing the marker of interest.

Tumour cells release naked DNA into the plasma as they necrose or apoptose, and the level of circulating free DNA correlates with disease burden. This can be used to monitor levels of tumour-specific point mutations or epigenetic changes in oncogenes with some prognostic value. However, the fraction of circulating naked DNA that derives from tumour cells is 0.01% or lower in many cases, and current methods for discriminating a single mutated base at this depth are inadequate. Thus, the existing strategies, which are based around point mutations or epigenetic changes in plasma DNA, suffer poor sensitivity and lack of specificity.

The present invention addresses the issue of detection and monitoring of diseases to support a personalized medicine approach.

### Statements of invention

The present invention relates to a method for monitoring a breast cancer, as set out in claim 1.

In a further aspect the invention relates to a method for assessing the efficacy of a treatment, as set out in claim 9.

### Figures

- Figure 1: illustrates quantitative detection of genome rearrangements in plasma DNA
- Figure 2: illustrates analysis of serial samples

### Detailed description

The present invention generally relates to detection of rearrangements in nucleic acid that are associated with a breast cancer state of a patient by genome wide analysis of the nucleic acid of that patient. Technologies now available allow the mapping of many nucleic acid breakpoints in a tissue sample, across a genome. Once suitable nucleic acid rearrangement biomarkers have been identified for a patient the progression of a disease may be followed by monitoring the increase or decrease in levels of nucleic acid containing said breakpoint(s) over time in that patient. Where the nucleic acid having the rearrangement is detectable in the blood or plasma, then blood or plasma samples may be sampled to easily monitor the progression disease. Treatments for the disease in a patient may be stopped once disease progression has halted, or been reversed, or is not detectable, as assessed by rearrangement biomarker levels. Treatment regimens may be altered or terminated if the disease burden is not reducing, as assessed by rearrangement biomarker levels. The effects of, and thus suitability of, different treatments can also be assessed..

The method of the invention may also be used to determine if relapse has occurred, to allow treatment to be restarted, if necessary.

In the example of cancer, rearrangement screens are potentially applicable to all tumour types in which a diagnostic sample can be accessed for genomic screening. Most patients with solid tumours undergo either biopsy or full surgical resection of their cancer during the course of their therapy, meaning that access to tumour DNA is usually achievable. In our experience to date, more than 99% of samples analysed, across a wide variety of tumour types, have had at least one identifiable tumour-specific genomic rearrangement.

Thus in a first aspect the invention relates to a method for monitoring a breast cancer as set out in claim 1.

A disease, as disclosed herein, may be any disease associated with a somatic rearrangement. Diseases may include, for example, cancers, such as solid tumours, Paroxysmal nocturnal hemoglobinuria, Neurofibromatosis 1 and 2, McCune-Albright, Incontinentia pigmenti, and Proteus syndrome.

Nucleic acid comprising the rearrangement is detectable in a sample from a body fluid such as blood, serum or, plasma.

Rearrangements associated with disease states herein, such as cancer, are not necessarily causative of that disease, although they may cause or contribute to the disease phenotype. However, it is only necessary that the rearrangement associates with the disease such that monitoring of the rearrangement can allow the progression or severity of the disease to be followed. Disclosed herein is monitoring using rearrangements that are not causative of disease, or not solely causative of disease.

Nucleic acid is taken directly from blood or plasma or serum, which is not itself known to be diseased, but from a patient known to have a disease. Somatically acquired genomic rearrangements are also useful as a marker of disease in such a case, where the assumption is made that the mutation is associated with the disease state.

The nucleic acid may be DNA, or RNA.

Genome wide analysis as disclosed herein is analysis of all or a significant part of the genome of an individual to identify mutations in the form of rearrangements that are found in diseased tissue of the individual, such as a tumour. Genome wide analysis is the identification of rearrangement mutations from an individual that correlate with disease by analysis of random nucleic acid fragments or regions from that individual, suitably without use of probes or primers that are known to be specific to nucleic acid from that individual. Thus it is not necessary to have complete coverage of a genome, although techniques that allow analysis of the whole genome, at least based upon a statistical analysis of coverage, are preferred.

Somatically acquired genomic rearrangements may include deletions, inversions, translocations and amplifications. The rearrangement may be detectable by a change in the length of a restriction fragment within which the mutation is located, in comparison with the patient's normal (non-mutated) genome.

In one aspect the analysis is carried out by sequencing DNA, for example, the sequencing of randomly generated fragments of the DNA of an individual. In one aspect the sequencing is sequencing of a library of sized DNA fragments, such as 400-500bp. In one aspect the technique used is massively parallel sequencing, as described herein, and also in Campbell et al Nature Genetics, Vol 40, number 6, June 2008, page 722 - 729.

Suitable massively parallel sequencing platforms also include the SOLiD platform (Applied Biosystems) and the use of the 454 sequencer (Roche).

In one aspect the sequencing is carried out using paired end sequencing. Suitably paired reads from in the order of 60 million fragments are generated, which is generally sufficient to identify >50% of somatic genomic rearrangements present in a sample.

Paired end sequencing methods are disclosed in, for example, Genome Res. 2009. 19: 521-532.

The genomic rearrangements may be prioritized. Prioritisation may be by, for example, including 1 or more of the following steps:
- ≥2 reads spanning the same rearrangement;
- High confidence mapping for both ends;
- Reads mapping <100kb apart on the same chromosome;
- Both ends mapping to within 100kb of a change-point in copy number identified by the segmentation algorithm.

The step of monitoring the changes in levels of nucleic acid containing the genomic rearrangement is a nested PCR approach. Reference herein to PCR generally refers to DNA amplification technologies, including the polymerase chain reaction specifically. Suitably primers designed to specifically identify the nucleic acid rearrangement are used in an amplification process.

In one aspect the PCR process is carried out on a nucleic acid sample obtained from blood, or serum.

In one aspect the size of the initial PCR product is less than < 200 bp, preferably less than 190 bp, 180 bp, 170 bp, 160 bp, 150 bp.

Suitably assays to monitor the level of a specific rearrangement are preferably substantially quantitative.

Changes in levels of the nucleic acid can be made by absolute or relative measurements. For example, the ratio of the level of 'normal' genomic DNA vs the mutated genomic DNA can be used. Alternatively, the absolute amount of mutated DNA can be measured, for example, DNA per ml of plasma. Measurement of the change in levels of nucleic acid, or quantification of levels of nucleic acid, can be either by ratio or absolute concentration measurement.

In one aspect the assay of the invention for monitoring changes in levels of nucleic acid in a patient in step is linearly quantifiable down to the level 25pg of DNA per assay.

In one aspect a one log increase in the absolute quantity of DNA detected with the rearrangement is considered to be a significant increase in disease burden, and may require treatment.

In one aspect of the invention multiple genomic rearrangements are monitored, to provide a genetic fingerprint of an individual.

Disclosed herein is a method of medical treatment comprising the monitoring of the invention, and additionally then comprising the step of treating the patient where necessary, or changing treatment, or stopping treatment if treatment is ongoing, depending upon disease severity or progression as indicated by the level of nucleic acid containing the informative rearrangement.

Disclosed herein is use of a patient specific genomic rearrangement as a biomarker for progression of disease in that patient.

It will be appreciated that the progression of a disease may be followed by monitoring the change in an identified marker over time. The severity of a disease may be assessed by a measurement at a single point in time of a biomarker whose presence or concentration is indicative of disease severity. For example, the presence in the blood of a somatic DNA rearrangement originally identified in a solid tumour may indicate the progression of a cancer beyond a certain disease state.

Disclosed herein is a method for determining a disease state, the method comprising identifying a somatically acquired genomic rearrangement associated with a disease state in a patient wherein the identification is carried out by genome-wide analysis of the nucleic acid of that patient. Suitably the genomic rearrangement is identified in a patient in a tissue or fluid, such as blood or plasma or serum. Preferably the genomic rearrangement is identified at a site, or in an organ tissue or fluid, other than in the primary diseased tissue from which the genome wide analysis was carried out.

For example, a solid tumour may represent a primary disease tissue, and the presence of a somatically acquired genomic rearrangement in the blood or other body fluid might be indicative of a certain disease progression of the cancer, and allow a therapeutic treatment to be determined.

Disclosed herein is a method for determining a treatment regimen for a disease, the method comprising quantifying the level of a somatically acquired genomic rearrangement associated with a disease state in a patient in a tissue or fluid, preferably other than in the primary diseased tissue, wherein the identification is carried out by genome-wide analysis of the nucleic acid of that patient, and selecting a treatment regimen based upon the level of said genomic rearrangement.

Disclosed herein is a method for assessing the efficacy of a treatment, the method comprising:
i Identifying a somatically acquired genomic rearrangement associated with a disease state in a patient, wherein the identification is carried out by genome-wide analysis of the nucleic acid of that patient, and
ii monitoring the changes in levels of nucleic acid containing the genomic rearrangement as a marker for the progression of a disease in that patient in response to a treatment, thereby assessing the efficacy of the treatment.

The treatment may be a novel treatment, in which case the method of the invention may not only be used to monitor the best treatment for a patient, it can also be used to generally determine the efficacy of new treatment regimens and new drug or other therapeutic treatments. This application is not limited to humans, but could also be applied to animals. Disclosed herein is a method for assessing the efficacy of a drug or treatment regimen, the method comprising treatment of an individual in need thereof with the drug or treatment regimen and then monitoring the progression or severity of disease after treatment by measurement of levels of nucleic acids with somatic rearrangements as a biomarker for disease.

Disclosed herein is a method for monitoring cell killing, wherein the killing of cells using a drug or treatment regimen is monitored by the release of nucleic acid comprising a somatic rearrangement.

Cancer Res 2007; 67: (19). October 1, 2007 p9364 - 9370 discloses monitoring of cell killing in an animal model.

If a patient is being treated with a drug or other therapy, for example, surgery, or radiotherapy or chemotherapy, then the effectiveness of that treatment can be monitored by looking for levels of the nucleic acid having the rearrangement in the patient.

Suitable treatments include the use of surgery, chemotherapy, radiotherapy, monoclonal antibodies, hormonal therapy and molecularly targeted therapy or a combination thereof.

In addition, where a patient has been treated for a disease and is in remission, then the continued remission status of the patient can be monitored. Should the patient come out of remission (relapse), then treatment can be restarted. Thus monitoring the progression of disease, as referred to herein, also includes monitoring the reoccurrence of disease after remission, and optionally treatment of the disease after relapse. The present invention may be used to monitor relapse after a period of, for example, hours, days, weeks or months after remission or the last cycle of treatment.

Disclosed herein is a method for determining the progression of cancer, the method comprising:
a) identifying a somatically acquired genomic rearrangement in nucleic acid from a tumour sample of a patient by genome-wide analysis through paired-end sequencing;
b) designing a quantitative assay for identifying and measuring the somatically acquired genomic rearrangements;
c) obtaining one of more further samples from the patient during future stages of therapy;
d) using the assays to measure levels of nucleic acids with somatic rearrangements in the samples obtained during step (a) and step (c); and optionally
e) determining the progression and/or severity of disease for the patient by comparing the levels of nucleic acids with somatic rearrangement measured in step (d).

As disclosed herein, pregnant women may be screened to determine whether hereditable diseases have been passed on to their children. Where a father is known to have a condition that is associated with a somatically acquired genomic rearrangement, the presence of this rearrangement can be assessed in, for example, the blood or serum, of the mother. Disclosed herein is a method suitable for monitoring a disease, the method comprising;
a identifying a somatically acquired genomic rearrangement associated with a disease state in a patient, wherein the identification is carried out by genome-wide analysis of the nucleic acid of that patient, and
b monitoring the changes in levels of nucleic acid containing the genomic rearrangement, and/or quantifying the levels of nucleic acid containing the genomic rearrangement, in a pregnant woman as a marker for the progression or severity of a disease in the foetus.

For the avoidance of doubt the terms 'comprising', 'comprise' and 'comprises' herein is intended by the inventors to be optionally substitutable with the terms 'consisting of', 'consist of', and 'consists of', respectively, in every instance. The term "about" (or "around") in all numerical values allows for a 5% variation, i.e. a value of about 1.25% would mean from between 1.19%-1.31%.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the measurement, the method being employed to determine the value, or the variation that exists among the study subjects. As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein.

The present invention is now exemplified with reference to the following examples, which are not limiting upon the invention

### Methods

The following steps are employed in this example of the invention
1. Identification of tumour-specific genomic rearrangements by massively parallel sequencing.
2. Mapping rearrangements to base-pair resolution.
3. Design of PCR-based assays for sensitive and specific quantification of tumour burden
4. Extraction of free DNA from serum and quantification of tumour load with appropriate controls.

### Identification of tumour-specific rearrangements by massively parallel sequencing

Genomic DNA is extracted from the tumour sample using phenol-chloroform extraction or other standard protocols. Libraries are prepared from tumour DNA according to the protocol recommended by the manufacturer of the massively parallel sequencing platform to be used. For the Solexa sequencing platform (Genome Analyzer, Illumina, San Diego CA), genomic DNA (5µg) is randomly sheared using the nebuliser supplied with the Genome Analyzer instrument according to the manufacturer's instructions. The fragmented DNA is end-repaired using T4 DNA polymerase and Klenow polymerase with T4 polynucleotide kinase to phosphorylate the 5' ends. A 3' A overhang is created using a 3'-5' exonuclease-deficient Klenow fragment, and Illumina paired-end adapter oligonucleotides are ligated to the sticky ends thus created. The ligation mixture is electrophoresed on an agarose gel, and size-selected by excising the DNA fragments of 400-500 base pairs in length. DNA is extracted from the gel and enriched for fragments with Solexa primers on either end by a limited cycle PCR reaction, following the manufacturer's instructions.

The Genome Analyzer paired-end flow-cell is prepared on the supplied cluster station according to the manufacturer's protocol. Clusters of PCR colonies are then sequenced on the Genome Analyzer platform using recommended protocols from the manufacturer. Paired-end sequencing of at least 35bp from either end gives the optimal coverage for identifying rearrangements, although they can be found with longer read-length, single-end reads. Images from the instrument are processed using the manufacturer's software to generate FASTQ sequence files.

Most of the current generation of massively parallel sequencing platforms can be used to identify genomic rearrangements, including the SOLiD platform (Applied Biosystems) and the 454 sequencer (Roche). Longer insert sizes increase coverage, and allow greater confidence in recognising clusters of rearrangements. Where sequencing data from the constitutional (germline) DNA from the same patient is available, this can be used to aid the distinction between germline and somatic rearrangements.

Sequence data are aligned to the reference human genome, using any of several freely available packages. We use the MAQ algorithm v0.4.3 (available at http://maq.sourceforge.net/maq-man.shtml). Reads in which the two ends failed to align to the genome in the correct orientation and distance apart are further screened with the SSAHA algorithm.

### Removal of artefacts

Reads where the two ends map back to within 500bp of one another, but with one of the two ends in the incorrect orientation are excluded from analysis, since they are likely to be artefacts due to either mis-priming within the PCR colony or intra-molecular rearrangements generated during library amplification. Reads which are exact duplicates of one another (created during the PCR enrichment step) are identified by the fact that the two ends of the sequences map to identical genomic locations: only the fragment with the higher mapping quality is retained. Spurious mapping of DNA from sequence gaps in the reference genome is reduced by excluding regions within 1 Mb of a centromeric or telomeric sequence gap from copy number and rearrangement analyses (see a list of current sequence gaps, for example, at: http://genome.ucsc.edu/cgi-bin/hgTables).

### Copy number algorithm

To correct for varying levels of uniqueness across the genome, an *in silico* simulation of paired-end short reads is performed by creating paired sequences of 35 bases each end, 500bp apart (or the equivalent if different libraries have been used), with simulated pairs located every 35bp along the genome. These simulated reads are mapped to the genome using the MAQ algorithm. On the basis of this, the genome is divided into non-overlapping, unequal width windows which contain a constant number of *in silico* reads mapped with high uniqueness. With the window boundaries set, the number of paired-end reads mapping uniquely within each window is counted. This forms the raw input to a binary circular segmentation algorithm originally developed for genomic hybridisation microarray data. This algorithm, implemented in R as the DNAcopy library of the Bioconductor project (see http://www.bioconductor.org/), identifies change-points in copy-number by iterative binary segmentation. We use α = 0.01, together with a smoothing parameter of 2 and 2 standard deviations for pruning of probable false positives after segmentation, although the modelling generally gives similar results for different parameter choices.

### Mapping rearrangements to base-pair resolution

The following criteria are used for prioritising incorrectly mapping reads for confirmatory screening:
1. ≥2 reads spanning the same rearrangement;
2. High confidence mapping for both ends;
3. Reads mapping <100kb apart on the same chromosome;
4. Both ends mapping to within 100kb of a change-point in copy number identified by the segmentation algorithm.

Primers are designed to span the possible breakpoint by locating them in the 1 kb outside the paired-end reads, for a maximum product size of 1 kb. PCR reactions are performed on tumour and normal genomic DNA for each set of primers. Products giving a band are sequenced by conventional Sanger capillary methods and compared to the reference sequence to identify breakpoints. Somatically acquired, tumour-specific rearrangements are defined as those PCR reactions giving a convincing band in the tumour DNA with no matching band in the normal DNA, seen in at least two separate reactions, together with unambiguously mapping sequence data suggesting a rearrangement.

Alternatively, rearrangements can be mapped by *de novo* assembly across the breakpoint. This is accomplished by extracting paired-end reads where one end maps in the location of the breakpoint. These reads can be assembled into longer contigs, which can be aligned against the reference genome to identify the exact location of the breaks.

### Design of PCR-based assays for quantification of tumour-specific rearrangements

We have found nested PCR to be a sensitive and specific method for amplification of tumour-specific genomic rearrangements.

Each confirmed somatic structural rearrangement is assessed for suitability as a DNA marker:

### Copy number change

It is important to select DNA markers present in the majority of the tumour cells. Rearrangement screens are carried out on tumour DNA derived from a population of cells, therefore the output of the sequencing experiment is a tumour cell population average of rearrangement. We seek to use rearrangements that i) are prevalent in the sequencing data and ii) have clear copy number changes, since these will be present in the majority (or all) of tumour cells.

### Uniqueness of surrounding DNA

Each assay must be specific for a particular rearrangement. The repetitive nature of the genome means that non-unique, repetitive sequences are located at multiple positions across the genome. In order to obtain highly specific assays the repetitive sequences surrounding each breakpoint are masked using repeat masker software (www.repeatmasker.org). This excludes a proportion of somatically acquired breakpoints from further analysis because of closely surrounding repeats. For some breakpoint junctions, no or only short stretches of nucleotides are masked out. This allows specific assays to be designed for these rearrangements if repeat sequences are avoided.

### Number of assays

We aim to design probes to 3-4 tumour-specific rearrangements per patient. Having multiple assays per patient increases confidence in the final result, although it is not always possible to identify this number of suitable rearrangements in every patient. Tumour specific assays are run alongside 4 control assays that are designed to recognise wild-type regions of the genome.

### Assay / oligo design

We take a nested PCR approach to identify cancer-specific rearrangements from the high background of circulating DNA derived from normal cells. Primers are designed using primer 3 (http://frodo.wi.mit.edu/) to avoid repetitive sequence and span the rearrangement breakpoint. The size of the initial PCR product should be kept to a minimum (< 200 bp) because circulating tumour DNA tends to be most abundant in this size range. The sequence amplified in the 1^{st} round of PCR is used as a template to design a dual labelled DNA probe ("taqman") style quantitative PCR assay using Beacon Designer software (Premier Biosoft International). This program selects primers and a dual labelled [5' FAM, 3' BHQ1] DNA probe. Due to the strict size constraints, overlap between the real time and 1^{st} round primers is sometimes required.

### Extraction of DNA from plasma/serum and quantification of tumour burden

### DNA extraction

Debris are removed from patient plasma or serum by centrifugation at 16, 000 g for 10 minutes. DNA is extracted from 2-20mL of the resultant supernatant using the QIAamp MinElute Virus Vacuum Kit (Qiagen). DNA is eluted in 20µl of the supplied elution buffer and the entire volume is used as template in the following PCR.

### Multiplex PCR

The entire quantity of DNA extracted from 2-20mL of patient plasma (or 10 fold serial dilution derived from this) is combined with all patient specific 1^{st} round PCR primers along with 1^{st} round control region primers and subjected to 20 cycles in a multiplex PCR. Combining all primers ensures that the highest possible quantity of DNA is available to each primer set.

### Real time PCR

Ten-fold dilutions were made of the nested PCR product and 5µl is used as template in individual real time PCR reactions using rearrangement specific primers and probes.

### Quantification

Using serial dilutions of the patient's tumour DNA in normal DNA (or water) allows production of a standard curve, since the amount (in pg) of tumour DNA per reaction is known. Using the curve of best fit applied to the standards then allows interpolation of the amount of tumour DNA present in the known volume of plasma/serum. In our experience, the nested, real-time PCR is capable of detecting down to 1 copy of the target rearrangement present in the whole volume of plasma analysed.

### Results

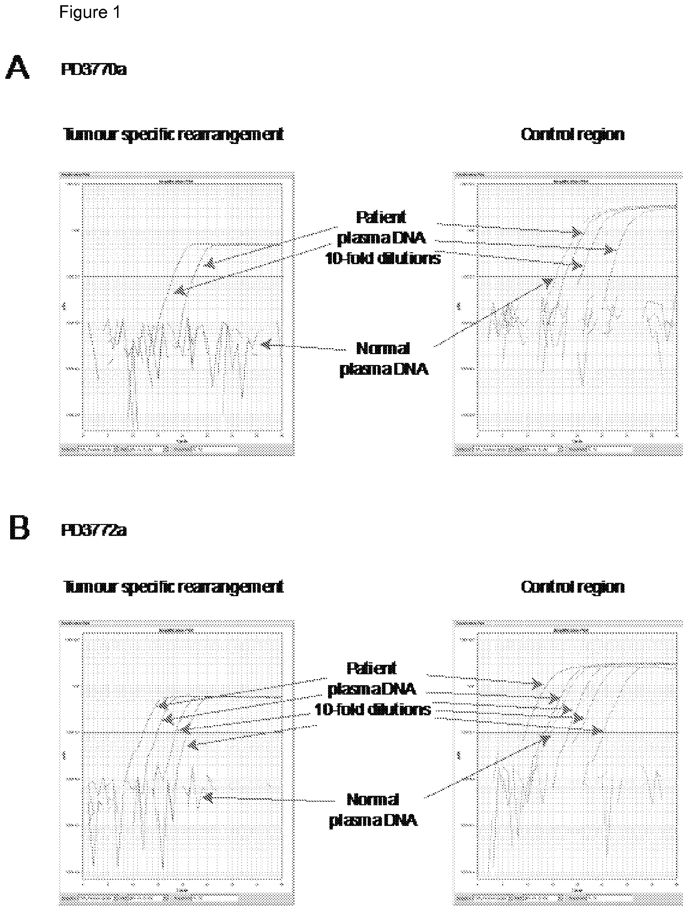

We investigated two patients with metastatic breast cancer. Massively parallel paired-end sequencing was used to identify somatically acquired genomic rearrangements from the genomes of both primary cancers and PCR assays were designed to amplify across multiple rearrangements from each genome (see Figure 1 below) The PCR products were sequenced to identify the breakpoints to base-pair resolution. We then designed nested real-time PCR assays to amplify and quantify the amount of tumour DNA.). After confirming the success of the PCR design on DNA from the cancer we then examined plasma samples taken at first presentation of disease in both cases. DNA was extracted from 2mL of plasma and analysed by the patient-specific real-time assays we designed. Figure 1 shows the results from the real-time PCR reactions. The curves show the amount of fluorescence generated by the real-time (Taqman) probes on the y axis with the number of PCR cycles on the x axis. The dark horizontal line around the middle of each figure 1 graph marks the level of fluorescence at which a reaction is deemed to reach positivity, so that the earlier (more leftward) a curve crosses the threshold, the greater the amount of target DNA in the reaction. Curves derived from plasma DNA from a normal individual (the negative control for the reaction) and curves showing serial 10-fold dilutions of the patient's plasma in water are identified by separate arrows in Figure 1. The left-hand graph for each patient shows the results for tumour-specific rearrangements. Clearly, the patient plasma samples are positive, whereas the negative control plasma (from a normal individual) is negative throughout. The right-hand graph for each patient shows the result for a normal region of the genome (positive control), which, as expected, is positive in both the patient and the normal control.

Figure 1: Quantitative detection of genomic rearrangements in plasma DNA of patients with breast cancer: somatically acquired genomic rearrangements were identified in the primary breast cancers of two patients with metastatic disease by massively parallel sequencing. DNA extracted from 2mL of plasma taken at diagnosis and serial 10-fold dilutions were screened by nested PCR with a final round of real-time PCT Robust detection of rearrangements (1 shown and 2 others not shown for each patient) was possible in both patients. Comparison of tumour-specific and control reactions in the dilution series suggests that in each patient the ratio of tumour-specific DNA to total DNA in plasma is 1:10, the total amount of plasma DNA extracted was ∼100x greater for patient PD3722a than PD3770a.

The results show that these somatic rearrangements can be quantitatively detected in plasma DNA. In particular, the following key features of the assay can be demonstrated from the analyses:
- The assay is highly sensitive, since the analyses were positive even with dilution of the plasma (1:10 dilution for the first patient, and 1:1000 for the second patient, equivalent to detecting a signal in the DNA from just 2µL of plasma, since 2mL was the starting volume).
- The assay is highly specific, since normal plasma DNA did not reveal a signal, even with nested PCR.
- The assay is quantitative, since the dilutions of plasma showed linear increases in the Cₜ, with robust separation between curves.

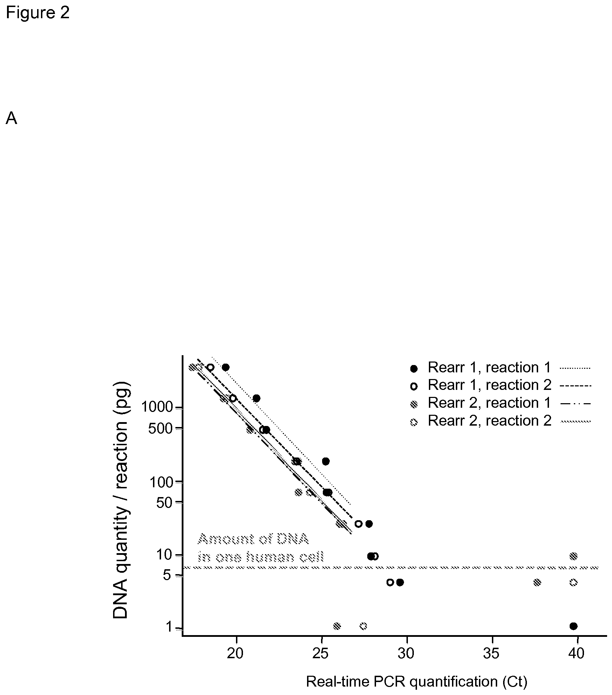

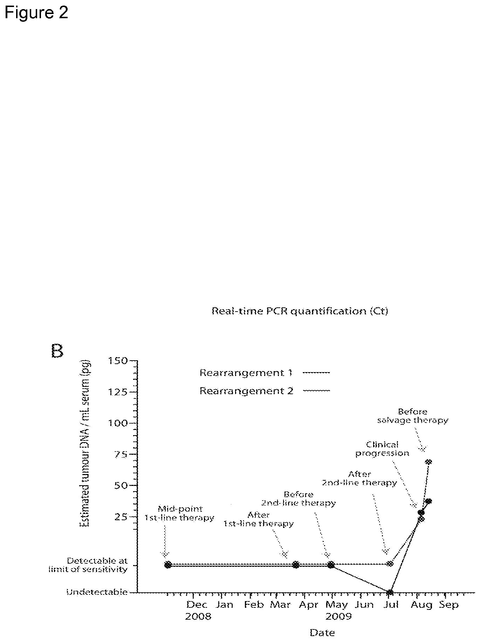

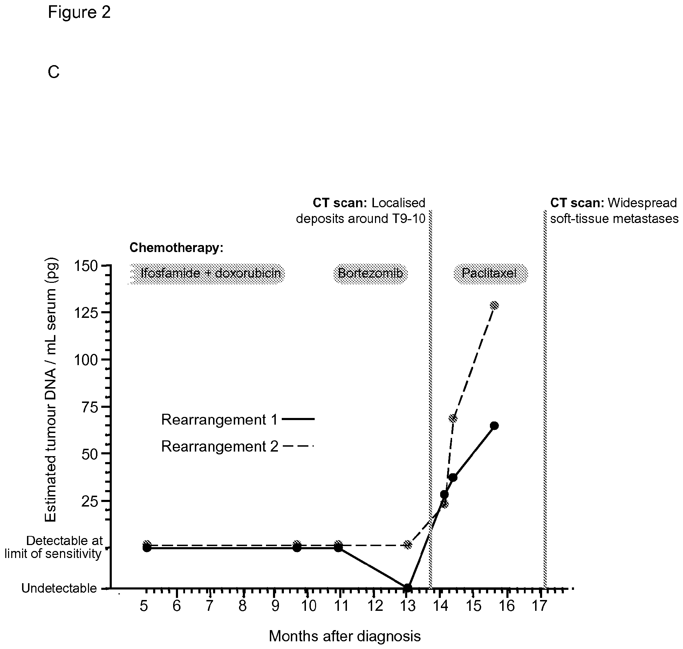

We have subsequently analysed serial samples from a third patient with cancer undergoing chemotherapy (Figure 2 below). As we did for the first two patients, a genome-wide rearrangement screen was undertaken using massively parallel sequencing to identify somatically acquired rearrangements. Two of these were selected for assay design. Serial dilutions of tumour DNA were made in normal DNA.

Figure 2A shows analysis of rearrangements 1 and 2 in duplicate reactions across a dilution series of tumour DNA into normal DNA. Where Ct≤27, the absolute amount of tumour DNA can be estimated from the line of best fit. For Ct>27, disease can only be classified as detectable or undetectable. However, the assay appears able to detect a single copy of the rearrangement present in the reaction. Figure 2B shows the estimated amount of tumour DNA per mL of serum from 6 samples collected at milestone time points in the patient's clinical course.

Panel A of figure 2 shows testing of the assays in replicate experiments on the serial dilutions. The results demonstrate that the assays are robust, reproducible and linearly quantifiable down to about 25pg of DNA per reaction. Given that a diploid human cell contains ∼6.75pg DNA, this is equivalent to ∼4 genomes per reaction. With lower amounts of tumour DNA in the reaction (5pg and 10pg per reaction), we find that reactions are either positive or negative (shown as points to the far right of the graph). This implies that in the negative reactions, no copies of the rearrangement were present, whereas in the positive reactions, 1 or 2 copies were present. A major finding therefore is that the nested real-time PCR assay would be capable of detecting a single copy of the rearrangement present in many millilitres of blood.

We next screened serial serum samples from the patient collected at time-points during her chemotherapy (Figures 2B and 2C). Unfortunately, no samples were available from before starting therapy. However, from the mid-point of her first-line chemotherapy through to the end of second-line chemotherapy, residual disease was detectable in the serum at the limits of detection of the assay. She unfortunately suffered clinical progression within a month or two of completing chemotherapy, and this was associated with an increase in the levels of disease detectable in her serum. In the time to schedule salvage chemotherapy, the levels of disease increased further.

These serial analyses demonstrate that the assay is capable of detecting disease even when present in minimal amounts clinically, and that the quantification of disease burden correlates with disease progression.

### Future Studies

### Aims:

We intend to measure the prognostic significance of tumour-specific rearrangements quantified in plasma DNA for:
1. 100 patients with non-metastatic breast cancer treated in an adjuvant therapy setting;
2. 100 patients with stage III or advanced stage II colorectal carcinoma.

### Non-metastatic breast cancer

Breast cancer is responsible for 16% of cancer deaths in women in the UK. For patients without known distal metastases at diagnosis, treatment is generally delivered with curative intent, but relapse rates range between 20-40% within 5 years depending on localised nodal involvement, size of the primary tumour and oestrogen receptor status. Many questions remain unanswered regarding the best use of adjuvant therapy in this clinical setting, and an accurate method to quantify disease burden would be invaluable for establishing personalised treatment regimens and optimising treatment intensity and duration.

### Stage III and high-risk stage II colorectal cancer

Colorectal cancer is responsible for 10% of all cancer deaths in the UK. Nearly half of all patients present with high-risk stage II or stage III disease, and this group has an overall 5-year survival of 33%-75% depending on the extent of local bowel and nodal involvement. For this reason, methods to accurately stratify patients into risk categories on the basis of persistent disease post-surgery would be particularly useful for clinical management.

### Plan of investigation

### Patient recruitment and sample collection

Patients with early-stage breast cancer to be treated by surgery and adjuvant therapy will be enrolled into the trial. All such women are reviewed in a pre-surgery Oncology clinic, and it is here that they will be approached, consented and enrolled in the study. At surgery, the breast cancer sample will be taken by the research nurse to the Pathology lab, where a portion of the tumour not required for diagnostic purposes will be fresh-frozen for subsequent DNA extraction. Serial samples of 20mL plasma will be extracted and frozen at important milestones during the patient's cancer care pathway: pre-surgery; post-surgery (adjuvant therapy planning clinic); end of chemotherapy (for ER-negative patients) or during hormonal therapy (ER-positive); 6-monthly during follow-up; at clinical relapse. Samples will be collected for assessment of circulating tumour cells by immunological methods after surgery and at the end of chemotherapy. Normal DNA will be extracted from whole blood leukocytes.

Patients with stage III and high-risk stage II colorectal cancer undergoing resection of the primary tumour with curative intent will be enrolled into the trial. All such patients are managed by a specific multidisciplinary team, which includes surgeons, medical oncologists and specialist nurses, for the whole of their staging/diagnostic phases, surgery, adjuvant therapy and post-treatment follow-up. Patients will be consented and enrolled in the study at pre-surgery review. At surgery, the colorectal cancer sample will be taken by the research nurse to the Pathology lab, where a portion of the tumour not required for diagnostic purposes will be fresh-frozen for subsequent DNA extraction. Serial samples of 20mL plasma will be extracted and frozen at important milestones during the patient's cancer care pathway: pre-surgery; post-surgery (adjuvant therapy planning clinic); end of chemotherapy; 6-monthly during follow-up; at clinical relapse. Normal DNA will be extracted from whole blood leukocytes.

### Paired-end sequencing

Briefly, standard protocols as described above will be followed to generate libraries of 400-500bp fragments for shotgun sequencing using 37bp paired-end reads. These will be used for massively parallel sequencing in order to generate paired reads from 60 million fragments, which is sufficient in our experience to identify >50% of somatic genomic rearrangements present in the sample. Using established algorithms, we will prioritise rearrangements for confirmatory PCR and capillary sequencing of the breakpoint - this step includes PCR across the normal DNA sample from the patient to prove the rearrangement is somatically acquired.

### Quantification of tumour-specific rearrangements in plasma DNA

Initially, 4 somatically acquired rearrangements per tumour will be taken forward for assay design. These will be chosen on the basis of:
- Presence in majority of tumour cells - this is best estimated by taking rearrangements demarcating integral changes in copy number (allowing for normal cell contamination).
- Unique DNA present at the breakpoint - the absence of repeats in the PCR amplicons will improve specificity of the assay.
- Involvement of cancer genes, where possible - for example, deletions of *CDKN2A* or the first rearrangement in *ERBB2* amplification are more likely to be present in all cells, including those that ultimately relapse.

Assays will be initially based on a first round of 20 cycles of PCR with primers designed to amplify a product no greater than 200bp (due to the small size of circulating tumour DNA fragments), followed by a second nested round of real-time PCR with a Taqman probe. Dilution series of tumour DNA and control amplicons will be used to estimate the relative fraction of plasma DNA that derives from tumour cells, as well as the total amount (see figure for examples). We have found this approach to give accurate, reproducible, linear quantification.

DNA will be extracted in batches from frozen plasma samples using established protocols and analysed in batches with the quantitative standards described above. Amplification of normal control regions from the genome will be used to estimate the total amount of naked DNA that is present in the plasma. It is likely that we will express the quantification of tumour DNA as a fraction of total naked plasma DNA, although it may be possible to quantify circulating tumour DNA as an absolute concentration.

### Correlation with clinical outcome and power calculations

Statistical analysis will focus on three questions: Prognostic significance of individual measurements at milestone time-points (presentation, post-surgery, at completion of therapy); ability to quantify drug-induced cell kill by evaluation of transient increases and subsequent fall in tumour-specific plasma DNA; and feasibility of predicting impending relapse through identification of rising levels before clinical complications develop. Power calculations show that for comparison of two groups of 25 patients stratified on ctDNA estimation, a hazard ratio of 2.2 could be detected with 80% power (on the basis of a 60 month study with median disease-free survival of 30 months in the poorer prognosis group). Additional prognostic analyses will be possible with the data set, such as correlation of markers of overall genomic instability with outcome and association of particular patterns of genomic rearrangement with survival.

### Statements of Invention

A A method suitable for monitoring a disease, the method comprising;
   a) identifying a somatically acquired genomic rearrangement associated with a disease state in a patient, wherein the identification is carried out by genome-wide analysis of the nucleic acid of that patient, and
   b) monitoring the changes in levels of nucleic acid containing the genomic rearrangement, and/or quantifying the levels of nucleic acid containing the genomic rearrangement, as a marker for the progression or severity of a disease in that patient.
B A method according to statement A wherein the disease is a cancer.
C A method according to statement B wherein the disease is a solid tumour.
D A method according to any preceding statement wherein the nucleic acid analysis in (a) is carried out on biopsied or surgically resected tumour tissue.
E A method according to any preceding statement wherein the genome-wide analysis is carried out by sequencing DNA.
F A method according to any preceding statement wherein the genome wide analysis is a massively parallel sequencing method.
G A method according to statement E or statement F wherein the sequencing is carried out using paired end sequencing.
H A method according to any preceding statement wherein the monitoring step in (b) is a PCR assay.
I A method according to any preceding statement wherein the monitoring step in (b) is a carried out on nucleic acid from a body fluid.
J A method according to statement A additionally comprising the step of treating the patient where necessary, or stopping treatment of the patient where necessary, as indicated by the level or change in level of nucleic acid containing the genomic rearrangement.
K A method according to statement A additionally comprising the step of determining a treatment regimen for treating a patient based upon the level or change in levels of nucleic acid containing the genomic rearrangement.
L Use of a patient specific genomic rearrangement as a biomarker for progression of disease in that patient.
M Use of a monitoring process according to statement A in the assessment of the efficacy of a drug or other therapeutic treatment.
N Use of a monitoring process according to statement M in the assessment of the efficacy of surgical resection of cancer tissue.
O A method for monitoring a disease, the method comprising monitoring the changes in levels of nucleic acid containing a genomic rearrangement, and/or quantifying the levels of nucleic acid containing a genomic rearrangement, wherein the genomic rearrangement is a somatically acquired mutation associated with a disease progression or severity in a patient, and wherein the identification of the rearrangement has been carried out by genome-wide analysis of the nucleic acid of that patient.
P A method according to statement A or O wherein monitoring changes in levels of nucleic acid containing the genomic rearrangement, and/or quantifying the levels of nucleic acid containing the genomic rearrangement, are carried out on a sample from a patient in remission.
Q A method or use according to any preceding statement wherein multiple somatically acquired genomic rearrangements are monitored.
R A method of medical treatment, the method comprising treating with an appropriate treatment, or changing treatment of, or stopping treatment of, a patient in whom a change in level of nucleic acid containing a genomic rearrangement identified according to statement A or O has been identified.

## Claims

1. A method for monitoring a breast cancer, the method comprising:
monitoring the changes in levels of nucleic acid containing a genomic rearrangement, and/or quantifying the levels of nucleic acid containing a genomic rearrangement, wherein the genomic rearrangement is a somatically acquired mutation associated with breast cancer progression or severity in a patient, and wherein the identification of the rearrangement has been carried out by genome-wide analysis of the nucleic acid of that patient or is carried out by genome-wide analysis of the nucleic acid of that patient, and wherein said monitoring is a nested PCR assay carried out on nucleic acid from a body fluid, wherein said body fluid is blood, serum or plasma.

2. A method according to claim 1 wherein the nucleic acid analysis is carried out on biopsied or surgically resected tumour tissue.

3. A method according to any preceding claim wherein the genome-wide analysis is carried out by sequencing DNA.

4. A method according to any preceding claim wherein the genome wide analysis is a massively parallel sequencing method.

5. A method according to claim 3 or claim 4 wherein the sequencing is carried out using paired end sequencing.

6. A method according to claim 1 additionally comprising the step of treating the patient where necessary, or stopping treatment of the patient where necessary, as indicated by the level or change in level of nucleic acid containing the genomic rearrangement.

7. A method according to claim 1 additionally comprising the step of determining a treatment regimen for treating a patient based upon the level or change in levels of nucleic acid containing the genomic rearrangement.

8. A method according to claim 1 wherein the monitoring of the breast cancer is monitoring for breast cancer progression.

9. *In vitro* use of a monitoring method according to claim 1 in the assessment of the efficacy of a drug or other therapeutic treatment, such as in the assessment of the efficacy of surgical resection of cancer tissue.

10. A method according to claim 1 wherein monitoring changes in levels of nucleic acid containing the genomic rearrangement, and/or quantifying the levels of nucleic acid containing the genomic rearrangement, are carried out on a sample from a patient in remission.

11. A method or use according to any preceding claim wherein multiple somatically acquired genomic rearrangements are monitored.

## Patentansprüche

1. Verfahren zur Überwachung von Brustkrebs, wobei das Verfahren das Folgende umfasst:
Überwachen der Änderungen der Ausmaße der Nucleinsäure, die eine genomische Umordnung enthält, und/oder Quantifizieren der Ausmaße der Nucleinsäure, die eine genomische Umordnung enthält, worin die genomische Umordnung eine somatisch erworbene Mutation ist, die mit Brustkrebsprogression oder -schwere bei einem Patienten verbunden ist, und worin die Identifikation der Umordnung durch genomweite Analyse der Nucleinsäure dieses Patienten durchgeführt wurde oder durch genomweite Analyse der Nucleinsäure dieses Patienten durchgeführt wird und worin die Überwachung ein Nested-PCR-Test ist, der an Nucleinsäure einer Körperflüssigkeit durchgeführt wird, worin die Körperflüssigkeit Blut, Serum oder Plasma ist.

2. Verfahren nach Anspruch 1, worin die Nucleinsäureanalyse an biopsiertem oder operativ entferntem Tumorgewebe durchgeführt wird.

3. Verfahren nach einem der vorangegangenen Ansprüche, worin die genomweite Analyse durchgeführt wird, indem DNA sequenziert wird.

4. Verfahren nach einem der vorangegangenen Ansprüche, worin die genomweite Analyse ein massiv paralleles Sequenzierverfahren ist.

5. Verfahren nach Anspruch 3 oder Anspruch 4, worin das Sequenzieren unter Verwendung von Sequenzierung mit gepaartem Ende durchgeführt wird.

6. Verfahren nach Anspruch 1, das zusätzlich, wenn nötig, den Schritt des Behandeins des Patienten oder, wenn nötig, des Beendens der Behandlung des Patienten umfasst, wie durch das Ausmaß oder die Veränderung des Ausmaßes der Nucleinsäure, die eine genomische Umordnung enthält, angezeigt wird.

7. Verfahren nach Anspruch 1, das zusätzlich den Schritt des Bestimmens eines Behandlungsschemas zur Behandlung eines Patienten auf Basis des Ausmaßes oder der Veränderung der Ausmaße der Nucleinsäure, die eine genomische Umordnung enthält, umfasst.

8. Verfahren nach Anspruch 1, worin die Überwachung des Brustkrebses eine Überwachung der Brustkrebsprogression ist.

9. In-vitro-Verwendung eines Überwachungsverfahrens nach Anspruch 1 zur Beurteilung der Wirksamkeit eines Arzneimittels oder einer anderen therapeutischen Behandlung, wie z.B. in der Beurteilung der Wirksamkeit der operativen Entfernung von Krebsgewebe.

10. Verfahren nach Anspruch 1, worin die Überwachung der Ausmaße der Nucleinsäure, die die genomische Umordnung enthält, und/oder das Quantifizieren der Ausmaße der Nucleinsäure, die die genomische Umordnung enthält, an einer Probe von einem Patienten in Remission durchgeführt wird/werden.

11. Verfahren oder Verwendung nach einem der vorangegangenen Ansprüche, worin multiple somatisch erworbene genomische Umordnungen überwacht werden.

## Revendications

1. Procédé de surveillance d'un cancer du sein, le procédé comprenant les étapes consistant à :
surveiller les changements dans les niveaux d'acide nucléique contenant un réarrangement génomique et/ou quantifier les niveaux d'acide nucléique contenant un réarrangement génomique, dans lequel le réarrangement génomique est une mutation acquise de façon somatique associée à une progression ou à une gravité du cancer du sein chez un patient, et dans lequel l'identification du réarrangement a été effectuée par analyse à l'échelle génomique de l'acide nucléique de ce patient ou est réalisée par analyse à l'échelle génomique de l'acide nucléique de ce patient, et dans lequel ladite surveillance est un dosage par PCR nichée réalisé sur un acide nucléiques provenant d'un fluide corporel, dans lequel ledit fluide corporel est du sang, du sérum ou du plasma.

2. Procédé selon la revendication 1, dans lequel l'analyse d'acide nucléique est effectuée sur un tissu tumoral soumis à une biopsie ou à une résection chirurgicale.

3. Procédé selon l'une quelconque des revendications précédentes, dans lequel l'analyse à l'échelle du génome est réalisée par séquençage d'ADN.

4. Procédé selon l'une quelconque des revendications précédentes, dans lequel l'analyse à l'échelle du génome est un procédé de séquençage massivement parallèle.

5. Procédé selon la revendication 3 ou la revendication 4, dans lequel le séquençage est réalisé en utilisant un séquençage d'extrémités appariées.

6. Procédé selon la revendication 1, comprenant en outre l'étape consistant à traiter le patient si nécessaire, ou à arrêter le traitement du patient si nécessaire, comme indiqué par le niveau ou le changement de niveau d'acide nucléique contenant le réarrangement génomique.

7. Procédé selon la revendication 1, comprenant en outre l'étape de détermination d'un régime de traitement pour traiter un patient sur la base du niveau ou du changement des niveaux d'acide nucléique contenant le réarrangement génomique.

8. Procédé selon la revendication 1, dans lequel la surveillance du cancer du sein surveille la progression du cancer du sein.

9. Utilisation *in vitro* d'un procédé de surveillance selon la revendication 1 dans l'évaluation de l'efficacité d'un médicament ou d'un autre traitement thérapeutique, comme dans l'évaluation de l'efficacité de la résection chirurgicale de tissu cancéreux.

10. Procédé selon l'une quelconque des revendications précédentes, **caractérisées en ce que** la surveillance des changements dans les niveaux d'acide nucléique contenant le réarrangement génomique et/ou la quantification des niveaux d'acide nucléique contenant le réarrangement génomique sont effectuées sur un échantillon d'un patient en rémission.

11. Procédé ou utilisation selon l'une quelconque des revendications précédentes, dans lequel de multiples réarrangements génomiques acquis de façon somatique sont surveillés.
